(19) Europäisches Patentamt
European Patent Office
Office européen des brevets

(11) Veröffentlichungsnummer: **0 035 716**
**B1**

(12) # EUROPÄISCHE PATENTSCHRIFT

(45) Veröffentlichungstag der Patentschrift: **29.06.83**

(21) Anmeldenummer: **81101424.0**

(22) Anmeldetag: **27.02.81**

(51) Int. Cl.³: **C 07 D 491/048** //
(C07D491/048, 307/00, 221/00)

(54) **4-Alpha-Cycloamino-arylmethyl-6-methyl-1,3-dihydro-furo(3,4-c)-pyridin-7-ole, ihre Herstellung und Verwendung.**

(30) Priorität: **06.03.80 DE 3008522**

(43) Veröffentlichungstag der Anmeldung:
**16.09.81 Patentblatt 81/37**

(45) Bekanntmachung des Hinweises auf die Patenterteilung:
**29.06.83 Patentblatt 83/26**

(84) Benannte Vertragsstaaten:
**BE CH DE FR GB LI NL**

(56) Entgegenhaltungen:
***keine***

(73) Patentinhaber: **BASF Aktiengesellschaft,
Carl-Bosch-Strasse 38, D-6700 Ludwigshafen (DE)**

(72) Erfinder: **Boell, Walter, Dr., Hintergasse 35,
D-6701 Dannstadt-Schauernheim (DE)**

EP 0 035 716 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 4-Alpha-Cycloamino-arylmethyl-6-methyl-1,3-dihydro-furo(3,4-c)-pyridin-7-ole, ihre Herstellung und Verwendung

Die vorliegende Erfindung betrifft 4-α-Cycloamino-arylmethyl-6-methyl-1,3-dihydro-furo[3,4-c]-pyridin-7-ole, Verfahren zu deren Herstellung und deren Verwendung zur Herstellung von Pyridinyl-aminoalkylethern.

Pyridinyl-aminoalkylether der Formel IV

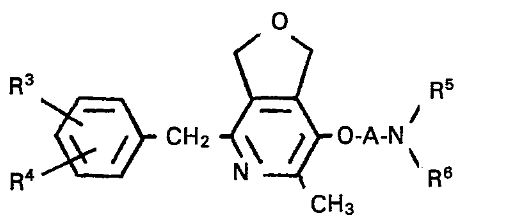

(IV)

worin

$R^3$ und $R^4$ Wasserstoff, Hydroxy, $C_{1-3}$-Alkyl, $C_{1-3}$-Alkoxy oder Halogen,

$R^5$ Wasserstoff, $C_{1-6}$-Alkyl, welches durch Hydroxy, $C_{1-4}$-Alkoxy, $C_{1-4}$-Alkylthio, $C_{1-4}$-Alkylamino, Di-$C_{1-4}$-alkylamino, oder einen gegebenenfalls durch $C_{1-3}$-Alkyl oder $C_{1-3}$-Alkoxy substituierten Phenoxy- oder Phenylring substituiert sein kann, $C_{3-6}$-Alkenyl, $C_{3-6}$-Alkinyl, $C_{3-8}$-Cycloalkyl, welcher durch $C_{1-3}$-Alkyl oder einen gegebenenfalls durch $C_{1-3}$-Alkyl oder $C_{1-3}$-Alkoxy substituierten Phenylring substituiert sein kann,

$R^6$ Wasserstoff oder $C_{1-6}$-Alkyl oder

$R^5$ und $R^6$ zusammen mit dem N-Atom einen 4- bis 8gliedrigen Ring, der ein weiteres Sauerstoff-, Stickstoff- oder Schwefelatom enthalten und durch $C_{1-3}$-Alkyl, $C_{3-8}$-Cycloalkyl, Hydroxy, $C_{1-4}$-Alkoxy oder einen gegebenenfalls durch $C_{1-3}$-Alkyl oder $C_{1-3}$-Alkoxy substituiertes Phenyl oder Phenyl-$C_{1-3}$-alkyl substituiert sein kann, und

A eine gegebenenfalls ungesättigte oder durch Hydroxy substituierte $C_{2-8}$-Alkylengruppe

darstellen, sind bereits aus der DE-OS 2 711 655 als antiarrhythmisch wirksame Verbindungen bekannt. Sie werden aus Oxazolen und Olefinen und anschliessende Umsetzung der so erhaltenen Pridinole mit Alkylierungsmitteln hergestellt. Dieses Verfahren ist recht umständlich, weil die als Ausgangsstoffe benötigten Oxazole nur schwer zugänglich sind.

Es wurden nun neue Zwischenprodukte gefunden, aus denen sich Pyridinyl-aminoalkylether auf einfache Weise darstellen lassen.

Gegenstand der Erfindung sind 4-α-Cycloamino-arylmethyl-6methyl-1,3-dihydro-furo[3,4-c]-pyridin-7-ole der Formel I

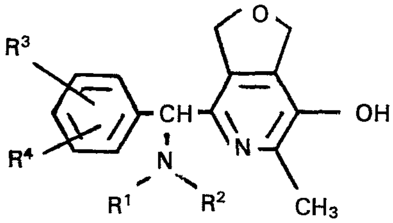

(I)

worin

$R^1$ und $R^2$ zusammen einen Alkylenrest, der durch ein Heteroatom unterbrochen und durch eine $C_{1-4}$-Alkylgruppe substituiert sein kann, und

$R^3$ und $R^4$ dasselbe wie oben

bedeuten.

Gegenstand der Erfindung ist weiter ein Verfahren zur Herstellung der Verbindungen der Formel I, welches darin besteht, dass man 6-Methyl-1,3-dihydro-[3,4-c]-pyridin-7-ol VI mit einem Aldehyd der Formel II

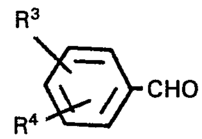

(II)

worin $R^3$ und $R^4$ dasselbe wie oben bedeuten, und einem cyclischen Amin der Formel III

$$HNR^1R^2 \qquad (III)$$

worin $R^1$ und $R^2$ dasselbe wie oben bedeuten, umsetzt.

Schliesslich betrifft die Erfindung ein Verfahren zur Herstellung von Verbindungen der Formel IV, welches darin besteht, dass man in einer Verbindung der Formel I die Cycloaminogruppe $-NR^1R^2$ hydrierend abspaltet und anschliessend in das Reaktionsprodukt die Gruppe der Formel V

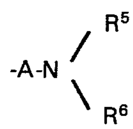

(V)

worin A, $R^5$ und $R^6$ dasselbe wie oben bedeuten, einführt.

Die Umsetzung von II mit III entspricht einer Mannich-Reaktion. Es gibt nur wenige Methoden, die es erlauben, in die 6-Position des Pyridoxin-Systems einen Substituenten einzuführen. Neben dem in der DE-OS 2 711 656 beschriebenen Weg ist ein erst kürzlich publiziertes Verfahren zu nennen (Y. Yamada et al., Tetrah. Letters 1979, 2603), das aber nicht zu den gewünschten Verbindungen führt. Eine Mannich-Reaktion am unsubstituierten oder niedrigsubstituierten Pyridin-3-ol ist bekannt. Sie führt jedoch zu den 2-substituierten Derivaten. Eine Substitution der 6-Position ist nur schwierig oder gar nicht zu erreichen [A. Stempel, E.C. Buzzi, J. Amer Chem. Soc. 71, 2969 (1949), J.V. Dejardin, C.L. Lapiere, Bull. Soc. chim. France II 1978, 75]. Es war deshalb überraschend und nicht vorhersehbar, dass im Fall des hochsubstituierten Verbindungstyps I die Reaktion in der beschriebenen Weise gelingt. Wie empfindlich die Reaktion von den Bedingungen abhängt, zeigt der Befund, dass nichtcyclische Amine wie Dipropyl-

amin, Diisobutylamin, N-Methylanilin oder Benzylamin nicht oder nur in untergeordnetem Mass die gewünschte Verbindung liefern.

Für die Umsetzung haben sich zwei Methoden als brauchbar erwiesen: Umsetzung aller Komponenten gleichzeitig (Methode A) oder primär Herstellung des Aminals aus Amin und Aldehyd und getrennte Umsetzung mit VI (Methode B), wobei in allen Fällen ein Unterschuss von VI als der teuersten Komponente zweckmässig ist. Man kann mit Lösungsmittel (Toluol, Chlorbenzol, Dimethylformamid, Dimethylsulfoxid, 1,2-Diethoxyethan) oder ohne Lösungsmittel arbeiten (in diesem Fall ist ein grosser Überschuss des Amins zweckmässig). Die Reaktionstemperatur soll zwischen 80 und 150°C liegen.

Als cyclische Amine sind vorzugsweise zu nennen:

| | |
|---|---|
| Piperidin | N-Methyl-piperazin |
| 3-Methylpiperidin | Hexamethylenimin |
| 4-Methylpiperidin | Pyrrolidin |
| Morpholin | 3-Methylpyrrolidin |
| 3-Methylmorpholin | 1,3-Propylenimin |
| Piperazin | Ethylenimin |

Bei der Umwandlung von I in IV wird zunächst der Rest $NR^1R^2$ in bekannter Weise hydrogenolytisch abgespalten (vgl. P.N. Rylander: Catalytic Hydrogenation in Organic Syntheses, Academic Press, New York 1979, Seiten 280-284). Als Katalysator benutzt man Palladium, Platin, Nickel- oder Kupferchromit. Bevorzugt ist Palladium, 5- bis 10%ig auf Aktivkohle aufgetragen, bei einem Wasserstoffdruck bis zu 50 bar und einer Reaktionstemperatur von 0 bis 60°C. Gebräuchliche Lösungsmittel sind Ester und Alkohole.

Die Umsetzung der so erhaltenen Verbindung mit V ist in der DE-OS 2 711 655 (vgl. Seiten 7-9) ausführlich beschrieben.

Durch die Erfindung wird es möglich, zur Synthese von IV von dem leicht zugänglichen Pyridoxin (Vitamin $B_6$) auszugehen, welches durch Einwirkung von Säure in 6-Methyl-1,3-dihydro-furo[3,4]-pyridin-7-ol VI übergeführt werden kann [vgl. J. Amer. Chem. Soc. 61, 3307 (1939)].

VI ist ein innerer Ether von Pyridoxin (Vitamin $B_6$) und besitzt folgende Struktur:

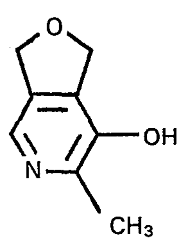

(VI)

*Beispiel 1* (Herstellung von I, Methode A)

a) Herstellung des Ausgangsmaterials

In einem 100-ml-Emailautoklav werden 50 g Pyridoxin-hydrochlorid (0,24 Mol) in die Mischung von 11 ml konzentrierte Salzsäure und 9 ml Wasser eingetragen. Man erhitzt 10 Stunden auf 150°C, lässt auf ca. 40°C abkühlen, saugt das Kristallisat ab und wäscht es mit 6 ml Ethanol nach. Nach dem Trocknen bei 100°C im Vakuum (20 mbar) erhält man 27,0 g 6-Methyl-1,3-dihydro-furo[3,4-c]-pyridin-7-ol-hydrochlorid; Schmp. 240-241°C.

b) Herstellung des Endprodukts

In einem 1-l-Vierhalskolben mit Rührer, Rückflusskühler, Thermometer und Tropftrichter werden unter Stickstoff 200 g Morpholin vorgelegt. Man trägt unter Rühren 75 g (0,4 Mol) des gemäss a) erhaltenen Produkts ein, lässt 64 g (0,6 Mol) Benzaldehyd zulaufen, wobei die Innentemperatur bis 95°C ansteigt, und erhitzt dann 2,5 Stunden auf 125 bis 130°C. Anschliessend wird im Vakuum (bis 25 mbar) bei 90 bis 100°C Morpholin abgezogen. In die auf 70°C abgekühlte zähe Mischung gibt man langsam 200 ml Methylenchlorid, dann unter Kühlung auf 20°C die Lösung von 66 g (1 Mol) 85%igem Kaliumhydroxid in 400 ml Wasser. Man rührt die beiden Phasen 5 Minuten kräftig, trennt sie und extrahiert die wässrig-alkalische Phase nochmals mit 100 ml Methylenchlorid. Die organischen Phasen werden verworfen. Die vereinigten wässrigen Lösungen werden mit 200 ml im Verhältnis 1:3 verdünnter konzentrierter Salzsäure auf pH 8 bis 9 eingestellt. Nach der Phasentrennung wird die wässrige Lösung nochmals mit 100 ml Methylenchlorid extrahiert. Man engt die Methylenchloridlösungen gemeinsam am Rotationsverdampfer ein und kristallisiert den Rückstand (149 g) aus 450 ml Isopropanol um. Die eisgekühlte, gut rührbare Suspension wird abgesaugt und mit wenig Isopropanol nachgewaschen. Nach dem Trocknen im Hochvakuum bei 60°C erhält man 141 g 4-(α-N-Morpholino)-benzyl-6-methyl-1,3-dihydro-furo[3,4-c]-pyridin-7-ol; Schmp. 83-85°C. Das Produkt enthält laut NMR-Spektrum und Elementaranalyse 2 Mol Isopropanol pro Mol Pyridinol und ist dünnschichtchromatographisch rein (Kieselgel, Essigester + 15% Methanol), Ausbeute 79%.

*Beispiel 2* (Herstellung von I, Methode B)

34,1 g (0,4 Mol) Piperidin werden unter Kühlung bei maximal 35°C zu 21,2 g (0,2 Mol) Benzaldehyd getropft. Das erstarrte Produkt wird aus Aceton umkristallisiert. Man erhält nach dem Trocknen 33,4 g Benzaldehyd-dipiperidinyl-aminal; Schmp. 80 bis 81°C.

Die Mischung aus 26 g (100 mMol) Benzaldehyd-dipiperidinyl-aminal und 12,1 g (80 mMol) 6-Methyl-1,3-dihydro-furo[3,4-c]-pyridin-7-ol in 60 ml Dimethylformamid wird 4 Stunden auf 110°C erhitzt. Bei 70°C/1,3 mbar wird der flüchtige Anteil abdestilliert. Durch Chromatographie des Rückstandes über Kieselgel (Methylenchlorid-Essigester) und Umkristallisation aus Aceton erhält man 13,0 g 4-(α-N-Piperidino)-benzyl-6-methyl-1,3-dihydro-furo[3,4-c]-pyridin-7-ol; Schmp. 169-170°C; Ausbeute 50%.

Analog erhält man folgende Substanzen:

| Beispiel | $R^3$ | $R^4$ | $N<^{R^4}_{R^5}$ | Herstellungsmethode | Ausbeute[1)] | Schmp. |
|---|---|---|---|---|---|---|
| 3 | H | H | Piperidin | A | 56% | 167-168°C |
| 4 | H | H | Pyrrolidin | A | 48% | 204-206°C[2)] |
| 5 | 4-$CH_3$ | H | Piperidin | A | 38% | 202-204°C[2)] |
| 6 | 4-Cl | H | » | A | 34% | 198-200°C[3)] |
| 7 | 3-$CH_3O$ | 4-$CH_3O$ | Morpholin | A | 49% | 172-173°C[3)] |

1) Produkt in einer für die Hydrogenolysestufe genügenden Reinheit.
2) als Monohydrochlorid.
3) als Bishydrochlorid.

*Beispiel 8*

Herstellung von (4-Benzyl-6-methyl-1,3-dihydro--furo[3,4-c]-pyridin-7-yl)-3-isopropylaminopropylether (vgl. Formel IV)

1. Stufe

In einem 0,5-l-Rührautoklav werden 67,5 g (150 mMol) 4-(α-N-Morpholino)-benzyl-6-methyl-1,3-dihydro-furo[3,4-c]-pyridin-7-ol (2 Mol Isopropanol pro Mol Pyridinol enthaltend) in 220 ml Methanol nach Zusatz von 1,5 g Pd/C (10%ig) bei 10 bar Wasserstoffdruck hydriert. Dauer der Wasserstoffaufnahme ca. 12 Stunden, Temperaturanstieg bis 35°C.

Nach 18 Stunden wird entspannt und mit Stickstoff gespült. Man setzt Natronlauge (6,6 g = 165 mMol NaOH in 47 ml Wasser) zu, rührt 20 Minuten, saugt den Katalysator ab und wäscht mit wenig Wasser nach. Das Filtrat (pH 12,8) wird mit 1/3 konz. Salzsäure auf pH 9 eingestellt, wobei das Produkt ausfällt, das bei 10°C abgesaugt und mit 120 ml Wasser nachgewaschen wird. Nach dem Trocknen bei 80°C/3 mbar bleiben 34,0 g 4-Benzyl-6-methyl--1,3-dihydro-furo[3,4-c]-pyridin-7-ol zurück, Schmp. 218-219°C; Ausbeute 94%.

2. Stufe

a) Die Mischung aus 24,2 g (100 mMol) 4-Benzyl-6-methyl-1,3-dihydro-furo[3,4-c]-pyridin-7-ol, 113 g (1 Mol) 1,3-Dichlorpropan, 2 g Benzyltriethylammoniumchlorid, 100 ml Toluol und 100 g 50%iger Natronlauge wird unter Rühren 3 Stunden auf 90°C erhitzt. Die organische Phase wird abgetrennt und mit 50 ml Wasser gewaschen. Nach dem Abziehen des Lösungsmittels und des überschüssigen 1,3-Dichlorpropans verbleiben 30,7 g (4-Benzyl-6-methyl--1,3-dihydro-furo[3,4-c]-pyridin-7-yl)-3-chlorpropylether mit kleinen, die folgende Umsetzung nicht störenden Anteilen an 1,3-Bis-(4-benzyl-6-methyl--1,3-dihydro-furo[3,4-c]-pyridin-7-oxy)-propan.

b) Die Mischung aus 24,2 g (100 mMol) 4-Benzyl--6-methyl-1,3-dihydro-furo[3,4-c]-pyridin-7-ol, 226 g (2 Mol) 1,3-Dichlorpropan, 2 g Benzyltriethylammoniumchlorid und 100 g 50%iger Natronlauge wird unter Rühren 3 Stunden auf 90°C erhitzt. Die organische Phase wird abgetrennt und mit 50 ml Wasser gewaschen. Nach dem Abziehen des überschüssigen 1,3-Dichlorpropans verbleiben 30,7 g (4-Benzyl--6-methyl-1,3-dihydro-furo[3,4-c]-pyridin-7-yl)-3--chlorpropylether mit kleinen, die folgende Umsetzung nicht störenden Anteilen an 1,3-Bis-(4-benzyl--6-methyl-1,3-dihydro-furo[3,4-c]-pyridin-7-oxy)--propan.

c) 24,2 g (100 mMol) 4-Benzyl-6-methyl-1,3-dihydro-furo[3,4-c]-pyridin-7-ol werden in 80 ml trockenem Dimethylsulfoxid suspendiert und durch Zugabe von 3,5 g (120 mMol) Natriumhydrid (85%ig in Öl) bei 20°C in das Natrumsalz übergeführt. Nach dem Ende der Wasserstoffentwicklung tropft man bei 0 bis 10°C 113 g (1 Mol) 1,3-Dichlorpropan zu und rührt 15 Stunden bei 20°C. Dimethylsulfoxid und überschüssiges 1,3-Dichlorpropan werden im Hochvakuum abdestilliert. Den Rückstand nimmt man in 250 ml Methylenchlorid auf, extrahiert zweimal mit 50 ml 10%iger Natronlauge und wäscht mit 10 ml Wasser. Nach dem Abdestillieren des Lösungsmittels verbleiben 30,7 g (4-Benzyl-6-methyl--1,3-dihydro-furo[3,4-c]-pyridin-7-yl)-3-chlorpropylether mit kleinen Anteilen an 1,3-Bis-(4-benzyl--6-methyl-1,3-dihydro-furo[3,4-c]-pyridin-7-oxy)--propan.

3. Stufe

8,0 g (25 mMol) des gemäss a), b) oder c) erhaltenen Produkts werden mit 15 g (250 mMol) Isopropylamin im Autoklaven 7 Stunden auf 100°C erhitzt. Das überschüssige Amin wird unter vermindertem Druck abdestilliert. Zur Reinigung des Rückstandes chromatographiert man über Kieselgel (Essigester, Methanol). Der anfallende (4-Benzyl-6-methyl-1,3--dihydro-furo[3,4-c]-pyridin-7-yl)-3-isopropylamino-propylether wird mit verdünnter Salzsäure in das Bis-hydrochlorid übergeführt und dieses aus Isopropanol umkristallisiert; 7,8 g, Schmp. 162°C.

Statt der Chromatographie kann zur Reinigung des Rohprodukts auch die Extraktion mit verdünnter Salzsäure dienen. Dazu gibt man unter Schütteln zur Lösung des Rohprodukts in Toluol so viel 1N-Salzsäure, bis die wässrige Phase einen pH-Wert von 6,7 aufweist. Durch Abtrennen und Einengen der wässrigen Phase erhält man (4-Benzyl-6-methyl-1,3-dihydro-furo[3,4-c]-pyridin-7-yl)-3-isopropylaminopropylether als Monohydrochlorid, während die schwächer basischen Verunreinigungen in der Toluolphase zurückbleiben.

*Beispiel 9*

12,1 g (50 mMol) 4-Benzyl-6-methyl-1,3-dihydro--furo[3,4-c]-pyridin-7-ol (vgl. Beispiel 8, Stufe 1) werden in 40 ml trockenem Dimethylsulfoxid suspendiert und durch Zugabe von 1,75 g (60 mMol) Natriumhydrid (85%ig in Öl) bei 20°C in das Natriumsalz überführt. Nach dem Ende der Wasserstoffentwicklung tropft man 8,1 g (75 mMol) frisch destilliertes β-Dimethylaminoethylchlorid zu und lässt 15 Stunden bei 10°C stehen. Das Dimethylsulfoxid wird im Hochvakuum abdestilliert. Man nimmt den Rückstand in Methylenchlorid auf, wäscht die Salze und nicht umgesetztes Pyridinol mit verdünnter Natronlauge aus, trocknet und engt im Vakuum ein. Der Rückstand wird mit verdünnter Salzsäure in das Hydrochlorid übergeführt und aus Ethanol-Ether umkristallisiert. Man erhält 10,0 g (4-Benzyl-6-methyl-1,3-dihydro-furo[3,4-c]-pyridin-7-yl)-β-dimethylaminoethylether-bis-hydrochlorid; Schmp. 213 bis 214°C.

*Beispiel 10*

12,1 g (50 mMol) 4-Benzyl-6-methyl-1,3-dihydro-furo[3,4-c]-pyridin-7-ol werden in 40 ml trockenem Dimethylsulfoxid suspendiert und durch Zugabe von 1,75 g (60 mMol) Natriumhydrid (85%ig in Öl) bei 20°C in das Natriumsalz übergeführt. Nach dem Ende der Wasserstoffentwicklung gibt man 9,25 g (100 mMol) Epichlorhydrin zu und erhitzt 1,5 Stunden auf 60°C, versetzt mit 30 g (0,5 Mol) Isopropylamin und erhitzt im Autoklaven 2,5 Stunden auf 100°C. Überschüssiges Amin und Dimethylsulfoxid werden im Vakuum abgezogen. Den Rückstand nimmt man in Methylenchlorid auf, wäscht die Lösung mit 10%iger Natronlauge und Wasser, trocknet und zieht das Lösungsmittel wieder ab. Der Rückstand wird zur Reinigung über Kieselgel chromatographiert (Essigester, Methanol). Nach Überführen in das Hydrochlorid und Umkristallisation aus Acetonitril erhält man 10,5 g (4-Benzyl-6-methyl-1,3-dihydro-furo[3,4-c]-pyridin-7-yl)-2-hydroxy-3-isopropylamino-propylether-hydrochlorid; Schmp. 152 bis 154°C.

Analog Beispiel 8 bis 10 werden die folgenden Verbindungen hergestellt (Schmelzpunkt der Hydrochloride):

11. (4-Benzyl-6-methyl-1,3-dihydro-furo[3,4-c]-pyridin-7-yl)-4-isopropylaminobuthylether; Schmp. 188-199°C.
12. (4-Benzyl-6-methyl-1,3-dihydro-furo[3,4-c]-pyridin-7-yl)-5-isopropylamino-(3-methylpentyl)-ether; Schmp. 169-170°C.
13. (4-Benzyl-6-methyl-1,3-dihydro-furo[3,4-c]-pyridin-7-yl)-5-diäthylamino-(3-methylpentyl)-ether; Schmp. 161-162°C.
14. (4-Benzyl-6-methyl-1,3-dihydro-furo[3,4-c]-pyridin-7-yl)-4-tert.-butylamino-butylether; Schmp. 198-199°C.

**Patentansprüche**

1. 4-α-Cycloamino-arylmethyl-6-methyl-1,3-dihydro-furo[3,4-c]-pyridin-7-ole der Formel I

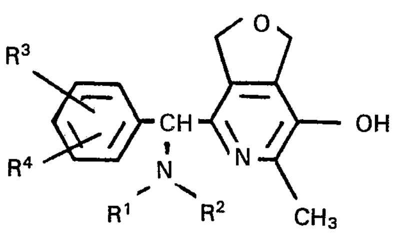

(I)

worin

$R^1$ und $R^2$ zusammen einen Alkylenrest, der durch ein Heteroatom unterbrochen und durch eine $C_{1-4}$-Alkylgruppe substituiert sein kann, und

$R^3$ und $R^4$ Wasserstoff, Hydroxy, $C_{1-3}$-Alkyl, $C_{1-3}$-Alkoxy oder Halogen

bedeuten.

2. Verfahren zur Herstellung der Verbindungen der Formel I gemäss Anspruch 1, dadurch gekennzeichnet, dass man 6-Methyl-1,3-dihydro-furo[3,4-c]-pyridin-7-ol mit einem Aldehyd der Formel II

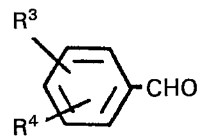

(II)

worin $R^3$ und $R^4$ dasselbe wie in Anspruch 1 bedeuten, und einem cyclischen Amin der Formel III

$$HNR^1R^2 \qquad \text{(III)}$$

worin $R^1$ und $R^2$ dasselbe wie oben bedeuten, umsetzt.

3. Verfahren zur Herstellung von Pyridinyl-aminoalkylethern der Formel IV

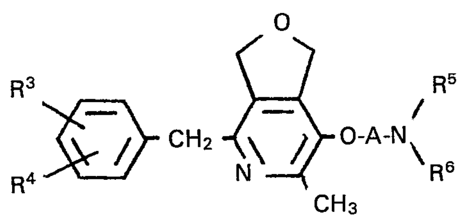

(IV)

worin

$R^3$ und $R^4$ Wasserstoff, Hydroxy, $C_{1-3}$-Alkyl, $C_{1-3}$-Alkoxy oder Halogen,

$R^5$ Wasserstoff, $C_{1-6}$-Alkyl, welches durch Hydroxy, $C_{1-4}$-Alkoxy, $C_{1-4}$-Alkylthio, $C_{1-4}$-Alkylamino, Di-$C_{1-4}$-alkylamino, oder einen gegebenenfalls durch $C_{1-3}$-Alkyl oder $C_{1-3}$-Alkoxy substituierten Phenoxy- oder Phenylring substituiert sein kann, $C_{3-6}$-Alkenyl, $C_{3-6}$-Alkinyl, $C_{3-8}$-Cycloalkyl, welcher durch $C_{1-3}$-Alkyl oder einen gegebenenfalls durch $C_{1-3}$-Alkyl oder $C_{1-3}$-Alkoxy substituierten Phenylring substituiert sein kann,

$R^6$ Wasserstoff oder $C_{1-6}$-Alkyl oder

$R^5$ und $R^6$ zusammen mit dem N-Atom einen 4- bis 8griedrigen Ring, der ein weiteres Sauerstoff-, Stickstoff- oder Schwefelatom enthalten und durch $C_{1-3}$-Alkyl, $C_{3-8}$-Cycloalkyl, Hydroxy, -$C_{1-4}$-Alkoxy oder einen gegebenenfalls durch $C_{1-3}$-Alkyl oder $C_{1-3}$-Alkoxy substituiertes Phenyl oder Phenyl-$C_{1-3}$-alkyl substituiert sein kann, und

A eine gegebenenfalls ungesättigte oder durch Hydroxy substituierte $C_{2-8}$-Alkylengruppe

darstellen, dadurch gekennzeichnet, dass man in einer Verbindung der Formel I die Cycloaminogruppe -$NR^1R^2$ gemäss Anspruch 1 hydrierend abspaltet und anschliessend in das Reaktionsprodukt die Gruppe der Formel V

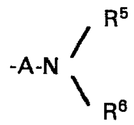

(V)

worin A, $R^5$ und $R^6$ dasselbe wie oben bedeuten, einführt.

**Claims**

1. A 4-α-cycloamino-arylmethyl-6-methyl-1,3-dihydro-furo[3,4-c]-pyridin-7-ol of the formula I

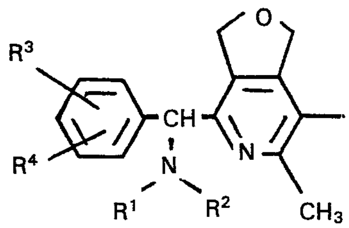

(I)

where

$R^1$ and $R^2$ together are alkylene which may be interrupted by a hetero-atom and may be substituted by a $C_{1-4}$-alkyl, and

$R^3$ and $R^4$ are hydrogen, hydroxyl, $C_{1-3}$-alkyl, $C_{1-3}$-alkoxy or halogen.

2. A process for the preparation of a compound of the formula I as claimed in claim 1, wherein 6-methyl-1,3-dihydro-furo[3,4-c]-pyridin-7-ol is reacted with an aldehyde of the formula II

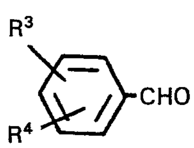

(II)

where $R^3$ and $R^4$ have the same meanings as in claim 1, and with a cyclic amine of the formula III

$HNR^1R^2$ (III)

where $R^1$ and $R^2$ have the same meanings as above.

3. A process for the preparation of pyridinyl aminoalkyl ethers of the formula IV

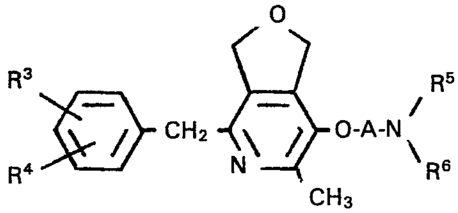

(IV)

where

$R^3$ and $R^4$ are hydrogen, hydroxyl, $C_{1-3}$-alkyl, $C_{1-3}$-alkoxy or halogen,

$R^5$ is hydrogen or $C_{1-6}$-alkyl, which may be substituted by hydroxyl, $C_{1-4}$-alkoxy, $C_{1-4}$-alkylthio, $C_{1-4}$-alkylamino or di-$C_{1-4}$-alkylamino or by phenoxy or phenyl which are unsubtituted or substituted by $C_{1-3}$-alkyl or $C_{1-3}$-alkoxy, or is $C_{3-6}$-alkenyl, $C_{3-6}$-alkynyl or $C_{3-8}$-cycloalkyl, which may be substituted by $C_{1-3}$-alkyl or by phenyl which is unsubstituted or substituted by $C_{1-3}$-alkyl or $C_{1-3}$-alkoxy,

$R^6$ is hydrogen or $C_{1-6}$-alkyl or

$R^5$ and $R^6$ together with the N atom are a 4-membered to 8-membered ring which may contain a further oxygen, sulphur or nitrogen atom, and may be substituted by $C_{1-3}$-alkyl, $C_{3-8}$-cycloalkyl, hydroxyl, $C_{1-4}$-alkoxy or phenyl or penyl-$C_{1-3}$-alkyl which are unsubstituted or are substituted by $C_{1-3}$-alkyl or $C_{1-3}$-alkoxy, and

A is $C_{2-8}$-alkylene which is saturated or unsaturated and unsubstituted or substituted by hydroxyl, wherein the cycloamino group $-NR^1R^2$ according to claim 1 is eliminated, by hydrogenation, from a compound of the formula I, and thereafter the group of the formula V

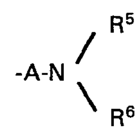

(V)

where A, $R^5$ and $R^6$ have the same meanings as above, is introduced into the reaction product.

**Revendications**

1. 4- -cycloamino-arylméthyl-6-méthyl-1,3-dihydro-furo[3,4-c]-pyridin-7-ols de formule I

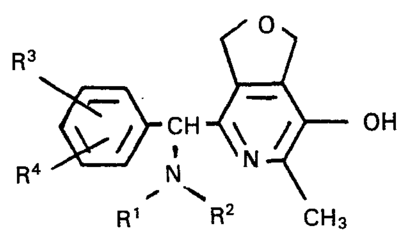

(I)

dans laquelle

$R^1$ et $R^2$ représente, ensemble, un reste alkyle, qui peut être entrecoupé par un hétéroatome et substitué par un groupe alkyle en $C_{1-4}$ et

$R^3$ et $R^4$ représentent hydrogène, hydroxy, alkyle en $C_{1-3}$, alcoxy en $C_{1-3}$ ou halogène.

2. Procédé de préparation des composés de formule I selon la revendication 1, caractérisé par le fait que l'on fait réagir du 6-méthyl-1,3-dihydro-furo-[3,4-c]-pyridin-7-ol avec un aldéhyde de formule II

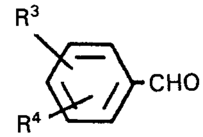

(II)

dans laquelle $R^3$ et $R^4$ représentent la même chose que dans la revendication 1, avec une amine cyclique de formule III

$HNR^1R^2$ (III)

où $R^1$ et $R^2$ représentent la même chose que ci-dessus.

3. Procédé de préparation de pyridinyl-aminoalkyl-ethers de formule IV

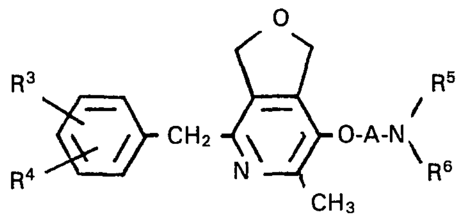

(IV)

où

$R^3$ et $R^4$ représentent hydrogène, hydroxy-alkyle en $C_{1-3}$, alcoxy en $C_{1-3}$ ou halogène,

$R^5$ hydrogène, alkyle en $C_{1-6}$ qui peut être substitué par hydroxy, alcoxy en $C_{1-4}$, alkylthio en $C_{1-4}$, alkylamino en $C_{1-4}$, di-alkylamino en $C_{1-4}$ ou un noyau phénoxy ou phényle éventuellement substitué par alkyle en $C_{1-3}$ ou alcoxy en $C_{1-3}$, alcényle en $C_{3-6}$, alcinyle en $C_{3-6}$, cycloalkyle en $C_{3-8}$ qui peut être substitué par alkyle en $C_{1-3}$ ou un noyau phényle éventuellement substitué par alkyle en $C_{1-3}$ ou alcoxy en $C_{1-3}$,

$R^6$ hydrogène ou alkylene en $C_{1-6}$, ou

$R^5$ et $R^6$ représentent ensemble avec l'atome N un noyau à 4 à 8 membres, qui contiennent un autre atome d'oxygène, d'azote ou de soufre et qui peut être substitué par alkyle en $C_{1-3}$, cycloalkyle en $C_{3-8}$, hydroxy, alcoxy en $C_{1-4}$ ou un phényle ou phénylealkyle en $C_{1-3}$ éventuellement substitué par alkyle en $C_{1-3}$ ou alcoxy en $C_{1-3}$,

A représente un groupe alkylène en $C_{2-8}$ éventuellement insaturé ou substitué par hydroxy, caractérisé par le fait que, dans un composé de formule I, on hydrogénolyse le groupe cycloamino $-NR^1R^2$ selon la revendication 1, puis on introduit, dans le produit de réaction, le groupe de formule V

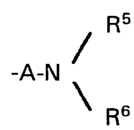

(V)

où A, $R^5$ et $R^6$ ont les mêmes significations que ci-dessus.